# EUROPEAN PATENT SPECIFICATION

(11) **EP 3 554 293 B1**
(45) Date of publication and mention of the grant of the patent: **03.02.2021**
(21) Application number: 17816652.6
(22) Date of filing: 07.12.2017
(51) Int. Cl.: A24F 40/10, A24F 40/30, A24F 40/42

(54) **AEROSOL-GENERATING SYSTEM HAVING AN EXTERNAL CARTRIDGE**
AEROSOLERZEUGUNGSSYSTEM MIT EXTERNER KARTUSCHE
SYSTÈME DE PRODUCTION D'AÉROSOL AYANT UNE CARTOUCHE EXTERNE

(30) Priority: 19.12.2016 EP 16205104
(43) Date of publication of application: 23.10.2019
(73) Proprietor: Philip Morris Products S.A., 2000 Neuchâtel (CH)
(72) Inventor: REEVELL, Tony, London EC2A 4NE (GB)
(74) Representative: Dowling, Ian
(86) International application number: PCT/EP2017/081931
(87) International publication number: WO 2018/114380

(56) References cited:
- WO-A1-2014/110119
- WO-A2-2016/135342
- US-A1- 2014 166 029

## Description

The present invention relates to an aerosol-generating system comprising an aerosol-generating device and a cartridge configured to be received on the aerosol-generating device. The invention finds particular application as an electrically operated smoking system.

One type of aerosol-generating system is an electrically operated smoking system. Known handheld electrically operated smoking systems typically comprise an aerosol-generating device comprising a battery, control electronics and an electric heater for heating an aerosol-forming substrate. The aerosol-forming substrate may be contained within part of the aerosol-generating device. For example, the aerosol-generating device may comprise a liquid storage portion in which a liquid aerosol-forming substrate, such as a nicotine solution, is stored. Such devices, often referred to as 'e-cigarettes', typically contain sufficient liquid aerosol-forming substrate to provide a number of puffs equivalent to consuming multiple conventional cigarettes.

US 2014/166029 A1 describes an e-cigarette comprising a battery section and a cartomizer, and various embodiments of a flavour enhancement that may be combined with the e-cigarette. In one embodiment the flavour enhancement is provided in the form of a tubular shell that is received within the cartomizer. In an alternative embodiment the flavour enhancement is in the form of a tubular shell that is received over an external wall of the cartomizer. The wall of the cartomizer may be formed from a permeable material or have holes extending therethrough to allow a flavouring agent from tubular shell to diffuse through the cartomizer wall and into the airflow inside the cartomizer.

In an attempt to provide e-cigarette users with an experience that more closely simulates the experience of consuming a conventional cigarette some devices have attempted to combine an e-cigarette configuration with a tobacco-based substrate to impart a tobacco taste to the aerosol inhaled by the user. However, to accommodate the tobacco-based substrate, such devices are typically significantly larger than convention e-cigarettes.

It would be desirable to provide an aerosol-generating system comprising multiple aerosol-forming substrates and which mitigates or eliminates at least some of these problems with known devices.

According to the present invention there is provided an aerosol-generating system as defined by claim 1, said system comprising a cartridge and an aerosol-generating device. The cartridge comprises a cartridge aerosol-forming substrate, the cartridge having an annular shape. The aerosol-generating device has a first end, a second end and a length extending between the first end and the second end. The aerosol-generating device comprises a liquid storage section comprising a liquid aerosol-forming substrate positioned within the liquid storage portion, and a first electric heater configured to heat liquid aerosol-forming substrate from the liquid storage section during use of the aerosol-generating system. The aerosol-generating device further comprises a power supply section comprising a power supply and a controller for controlling a supply of electrical power from the power supply to the first electric heater. The aerosol-generating device also comprises a device air inlet positioned between the first end and the second end of the device, and a device air outlet positioned at the second end of the device. The aerosol-generating device is configured to receive the cartridge on the aerosol-generating device so that the cartridge at least partially overlies the device air inlet.

As used herein, the term "aerosol-forming substrate" is used to describe a substrate capable of releasing volatile compounds, which can form an aerosol. The aerosols generated from aerosol-forming substrates of aerosol-generating systems according to the invention may be visible or invisible and may include vapours (for example, fine particles of substances, which are in a gaseous state, that are ordinarily liquid or solid at room temperature) as well as gases and liquid droplets of condensed vapours.

Aerosol-generating systems according to the present invention provide an annular cartridge configured to be received on an aerosol-generating device so that the cartridge overlies a device air inlet. Therefore, in use, air is drawn into the aerosol-generating device through the cartridge.

Advantageously, this arrangement eliminates the need for a cartridge-receiving cavity in the aerosol-generating device. Eliminating a cartridge-receiving cavity can facilitate an aerosol-generating device length that is similar to conventional e-cigarettes. Eliminating a cartridge-receiving cavity makes it easier for a user to engage and disengage the cartridge with the aerosol-generating device. In particular, the risk of a cartridge becoming stuck in a cartridge-receiving cavity is eliminated.

Advantageously, this arrangement facilitates the use of an aerosol-generating device that is similar to conventional e-cigarettes, which may reduce or eliminate the need to modify existing devices.

Preferably, the aerosol-generating system is configured so that the cartridge is retained on the aerosol-generating device by an interference fit.

An outer surface of the aerosol-generating device forms a flange against which the cartridge is received when the cartridge is received on the aerosol-generating device, wherein the device air inlet is positioned proximate the flange. Advantageously, providing a flange against which the cartridge is received may facilitate correct placement of the cartridge on the aerosol-generating device by a user.

The device air inlet may be positioned on the flange so that an end of the cartridge abuts the device air inlet when the cartridge is received against the flange.

The device air inlet may be positioned on the outer surface of the aerosol-generating device adjacent the flange so that an inner surface of the cartridge at least partially overlies the device air inlet when the cartridge is received against the flange.

The liquid storage section is removable from the power supply section. Advantageously, this facilitates replacement of the liquid storage section (for example, when the liquid aerosol-generating substrate has been depleted) without replacement of the power supply section. The liquid storage section may be configured for removable attachment to the power supply section by at least one of an interference fit, a screw connection and a bayonet connection.

The power supply section has a first end forming the first end of the aerosol-generating device and a second end, wherein the liquid storage section has a first end configured for removable attachment to the second end of the power supply section and a second end forming the second end of the aerosol-generating device. One of the first end of the liquid storage section and the second end of the power supply section forms the flange when the liquid storage section is attached to the power supply section. An outer dimension of the second end of the power supply section may be larger than an outer dimension of the first end of the liquid storage section. An outer dimension of the first end of the liquid storage section may be larger than an outer dimension of the second end of the power supply section.

The aerosol-generating system may further comprise a second electric heater provided on an outer surface of the aerosol-generating device, wherein the second electric heater is positioned so that the cartridge at least partially overlies the second electric heater when the cartridge is received on the aerosol-generating device. Preferably, the controller is configured to control a supply of electrical power from the power supply to the second electric heater when the cartridge is received on the aerosol-generating device. Advantageously, providing a second heater configured to heat the cartridge may facilitate the release of volatile compounds from the cartridge aerosol-forming substrate during use of the aerosol-generating system.

The second heater may comprise a substantially annular heater extending around a portion of the outer surface of the aerosol-generating device. The second heater may comprise a plurality of discrete heaters positioned around a portion of the outer surface of the aerosol-generating device.

The aerosol-generating system may further comprise a removable cover configured to receive at least a portion of the aerosol-generating device, wherein the removable cover is configured to overlie the cartridge when the cartridge is received on the aerosol-generating device and when the removable cover is engaged with the aerosol-generating device.

Advantageously, the removable cover may protect the cartridge when the aerosol-generating system is handled by a user during use. Preventing a user touching the cartridge during use may be particularly desirable in embodiments in which the aerosol-generating system comprises a second heater configured to heat the cartridge.

Advantageously, the removable cover in combination with the aerosol-generating device may provide the aerosol-generating system with at least one of a visual appearance and external dimensions that are substantially the same as a conventional e-cigarette. Advantageously, this may provide compatibility of the aerosol-generating system with existing accessories, such as a carry case.

Preferably, the removable cover has a substantially cylindrical shape. The removable cover may be substantially closed at one end. The removable cover may comprise a cover air outlet configured for fluid communication with the device air outlet when the removable cover is engaged with the aerosol-generating device. In embodiments in which the removable cover is substantially closed at one end, preferably the cover air outlet is provided on the closed end.

The removable cover may be comprise a cover air inlet configured for fluid communication with the cartridge when the cartridge is received on the aerosol-generating device and when the removable cover is engaged with the aerosol-generating device.

A first end of the removable cover may be configured to abut the flange when the removable cover is engaged with the aerosol-generating device. Advantageously, this may facilitate correct placement of the removable cover on the aerosol-generating device by a user.

Preferably, the removable cover is configured to be retained on the aerosol-generating device by an interference fit.

The cartridge may form a sleeve configured to slide onto a portion of the aerosol-generating device.

The cartridge may be configured to be received between the liquid storage section and the power supply section. A connecting portion of one or both of the liquid storage section and the power supply section may extend through the cartridge when the cartridge is received on the aerosol-generating device.

The cartridge may form an annular disc. The annular disc may be one of a ring shape, a split-ring shape, and a C-shape.

Advantageously, a ring shape may prevent the cartridge becoming detached from the aerosol-generating device while the liquid storage section is attached to the power supply section.

Advantageously, a split-ring shape may facilitate engagement of the cartridge with a connecting portion on the liquid storage section or the power supply section by allowing a small degree of expansion and contraction in the size of the ring.

Advantageously, a C-shape may facilitate engagement and disengagement of the cartridge with the aerosol-generating device without separating the liquid storage section from the power supply section. For example, in embodiments in which the liquid storage section removable attaches to the power supply section by a screw connection, engagement and disengagement of the cartridge with the aerosol-generating device may be facilitated by only partially unscrewing the liquid storage section from the power supply section.

The cartridge may comprise a porous wrapper overlying at least a portion of the cartridge aerosol-forming substrate. The porous wrapper may substantially encapsulate the cartridge aerosol-forming substrate. The porous wrapper may comprise at least one of a woven material and a non-woven material. The porous wrapper may comprise a mesh. The porous wrapper may comprise a paper. Advantageously, providing a porous wrapper overlying at least a portion of the cartridge aerosol-forming substrate may retain the cartridge aerosol-forming substrate in the desired shape and allow air to flow through the cartridge during use.

The cartridge may comprise a cartridge housing, wherein the cartridge aerosol-forming substrate is positioned within the cartridge housing. Preferably, the cartridge housing comprises a cartridge air inlet and a cartridge air outlet. The cartridge is configured so that, when the cartridge is engaged with the aerosol-generating device, the cartridge air outlet overlies the device air inlet. Each of the cartridge and the aerosol-generating device may comprise one or more indicia to indicate to a user the rotational orientation of the cartridge with respect to the aerosol-generating device. Advantageously, this may assist a user in aligning the cartridge air outlet with the device air inlet.

The cartridge housing may be formed from any suitable material or combination of materials. Suitable materials include, but are not limited to, aluminium, polyether ether ketone (PEEK), polyimides, such as Kapton®, polyethylene terephthalate (PET), polyethylene (PE), high-density polyethylene (HDPE), polypropylene (PP), polystyrene (PS), fluorinated ethylene propylene (FEP), polytetrafluoroethylene (PTFE), polyoxymethylene (POM), epoxy resins, polyurethane resins, vinyl resins, liquid crystal polymers (LCP) and modified LCPs, such as LCPs with graphite or glass fibres.

The aerosol-generating system may comprise a removable wrapper enclosing the cartridge. The removable wrapper is removed from the cartridge prior to use of the cartridge with the aerosol-generating device. Preferably, the removable wrapper comprises a non-porous material. The removable wrapper may comprise a polymeric material, such as a polymeric film. The removable wrapper may comprise a laminate of two or more different materials. The removable wrapper may comprise a metallised polymeric film.

The first electric heater may be provided separately from both the liquid storage section and the power supply section, wherein the first electric heater is configured for removable attachment to at least one of the liquid storage section and the power supply section.

The first electric heater may be provided integrally with one of the power supply section and the liquid storage section.

Preferably, the first electric heater and liquid storage section are provided together in a vaporiser section of the aerosol-generating device. In embodiments in which the liquid storage section is configured for removable attachment to the power supply section, the vaporiser section is configured for removable attachment to the power supply section.

The cartridge aerosol-forming substrate may comprise a solid aerosol-forming substrate. The solid aerosol-forming substrate may comprise tobacco. The solid aerosol-forming substrate may comprise a tobacco-containing material containing volatile tobacco flavour compounds which are released from the substrate upon heating.

The solid aerosol-forming substrate may comprise tobacco containing deprotonated nicotine. Deprotonating the nicotine within tobacco may advantageously increase the volatility of the nicotine. Nicotine may be deprotonated by subjecting the tobacco to an alkalising treatment.

The solid aerosol-forming substrate may comprise a non-tobacco material. The solid aerosol-forming substrate may comprise tobacco-containing material and non-tobacco containing material.

The solid aerosol-forming substrate may include at least one aerosol-former. As used herein, the term 'aerosol former' is used to describe any suitable known compound or mixture of compounds that, in use, facilitates formation of an aerosol. Suitable aerosol-formers include, but are not limited to: polyhydric alcohols, such as propylene glycol, triethylene glycol, 1,3-butanediol and glycerine; esters of polyhydric alcohols, such as glycerol mono-, di- or triacetate; and aliphatic esters of mono-, di- or polycarboxylic acids, such as dimethyl dodecanedioate and dimethyl tetradecanedioate

Preferred aerosol formers are polyhydric alcohols or mixtures thereof, such as propylene glycol, triethylene glycol, 1,3-butanediol and, most preferred, glycerine.

The solid aerosol-forming substrate may comprise a single aerosol former. Alternatively, the solid aerosol-forming substrate may comprise a combination of two or more aerosol formers.

The solid aerosol-forming substrate may have an aerosol former content of greater than 5 percent on a dry weight basis.

The solid aerosol-forming substrate may have an aerosol former content of between approximately 5 percent and approximately 30 percent on a dry weight basis.

The solid aerosol-forming substrate may have an aerosol former content of approximately 20 percent on a dry weight basis.

The liquid aerosol-forming substrate of the liquid storage section may comprise a tobacco-containing material comprising volatile tobacco flavour compounds which are released from the liquid upon heating. The liquid aerosol-forming substrate may comprise a non-tobacco material. The liquid aerosol-forming substrate may include water, solvents, ethanol, plant extracts and natural or artificial flavours. Preferably, the liquid aerosol-forming substrate comprises an aerosol former. Suitable aerosol formers include polyhydric alcohols or mixtures thereof, such as propylene glycol, triethylene glycol, 1,3-butanediol and glycerine.

The liquid aerosol-forming substrate in the liquid storage section may comprise nicotine.

The liquid aerosol-forming substrate may be free from nicotine.

The liquid storage section may comprise a porous carrier material, wherein the liquid aerosol-forming substrate is provided on the porous carrier material. Advantageously, providing the liquid aerosol-forming substrate on a porous carrier material may reduce the risk of the liquid aerosol-forming substrate leaking from the liquid storage section.

The porous carrier material may comprise any suitable material or combination of materials which is permeable to the liquid aerosol-forming substrate and allows the liquid aerosol-forming substrate to migrate through the porous carrier material. Preferably, the material or combination of materials is inert with respect to the liquid aerosol-forming substrate. The porous carrier material may or may not be a capillary material. The porous carrier material may comprise a hydrophilic material to improve distribution and spread of the liquid aerosol-forming substrate. This may assist with consistent aerosol formation. The particular preferred material or materials will depend on the physical properties of the liquid aerosol-forming substrate. Examples of suitable materials are a capillary material, for example a sponge or foam material, ceramic- or graphite-based materials in the form of fibres or sintered powders, a foamed metal or plastics material, a fibrous material, for example made of spun or extruded fibres, such as cellulose acetate, polyester, or bonded polyolefin, polyethylene, terylene or polypropylene fibres, nylon fibres or ceramic. The porous carrier material may have any suitable porosity so as to be used with different liquid physical properties.

The cartridge aerosol-forming substrate may comprise a liquid aerosol-forming substrate. The liquid aerosol-forming substrate may be provided on a porous carrier material positioned within the cartridge. Suitable liquid aerosol-forming substrates include those described herein with respect to the liquid storage section of the aerosol-generating device. Suitable porous carrier materials include those described herein with respect to the liquid storage section of the aerosol-generating device. Preferably, the liquid aerosol-forming substrate provided in the cartridge is different to the liquid aerosol-forming substrate provided in the liquid storage section of the aerosol-generating device.

The aerosol-generating system may further comprise a liquid transfer element configured so that, in use, liquid aerosol-forming substrate is transported by capillary action along the liquid transfer element from the liquid storage section to the electric heater. In embodiments in which the liquid storage section comprises a porous carrier material, the liquid transfer element is configured to transport liquid aerosol-forming substrate from the porous carrier material to the electric heater.

The liquid transfer element may comprise any suitable material or combination of materials which is able to convey the liquid aerosol-forming substrate along its length. The liquid transfer element may be formed from a porous material, but this need not be the case. The liquid transfer element may be formed from a material having a fibrous or spongy structure. The liquid transfer element preferably comprises a bundle of capillaries. For example, the liquid transfer element may comprise a plurality of fibres or threads or other fine bore tubes. The liquid transfer element may comprise sponge-like or foam-like material. Preferably, the structure of the liquid transfer element forms a plurality of small bores or tubes, through which the liquid aerosol-forming substrate can be transported by capillary action. The particular preferred material or materials will depend on the physical properties of the liquid aerosol-forming substrate. Examples of suitable capillary materials include a sponge or foam material, ceramic- or graphite-based materials in the form of fibres or sintered powders, foamed metal or plastics material, a fibrous material, for example made of spun or extruded fibres, such as cellulose acetate, polyester, or bonded polyolefin, polyethylene, terylene or polypropylene fibres, nylon fibres, ceramic, glass fibres, silica glass fibres, carbon fibres, metallic fibres of medical grade stainless steel alloys such as austenitic 316 stainless steel and martensitic 440 and 420 stainless steels. The liquid transfer element may have any suitable capillarity so as to be used with different liquid physical properties. The liquid aerosol-forming substrate has physical properties, including but not limited to viscosity, surface tension, density, thermal conductivity, boiling point and vapour pressure, which allow the liquid aerosol-forming substrate to be transported through the liquid transfer element. The liquid transfer element may be formed from heat-resistant material. The liquid transfer element may comprise a plurality of fibre strands. The plurality of fibre strands may be generally aligned along a length of the liquid transfer element.

In embodiments in which the liquid storage section comprises a porous carrier material, the porous carrier material and the liquid transfer element may comprise the same material. Preferably, the porous carrier material and the liquid transfer element comprise different materials.

The first electric heater may comprise a resistive heating coil.

The first electric heater may comprise a resistive heating mesh.

The resistive heating mesh may comprise a plurality of electrically conductive filaments. The electrically conductive filaments may be substantially flat. As used herein, "substantially flat" means formed in a single plane and not wrapped around or otherwise conformed to fit a curved or other non-planar shape. A flat heating mesh can be easily handled during manufacture and provides for a robust construction.

The electrically conductive filaments may define interstices between the filaments and the interstices may have a width of between about 10 micrometres and about 100 micrometres. Preferably the filaments give rise to capillary action in the interstices, so that in use, liquid aerosol-forming substrate is drawn into the interstices, increasing the contact area between the heater assembly and the liquid.

The electrically conductive filaments may form a mesh of size between about 160 Mesh US and about 600 Mesh US (+/- 10%) (that is, between about 160 and about 600 filaments per inch (+/- 10%)). The width of the interstices is preferably between about 75 micrometres and about 25 micrometres. The percentage of open area of the mesh, which is the ratio of the area of the interstices to the total area of the mesh is preferably between about 25 percent and about 56 percent. The mesh may be formed using different types of weave or lattice structures. The electrically conductive filaments may be an array of filaments arranged parallel to one another.

The electrically conductive filaments may have a diameter of between about 8 micrometres and about 100 micrometres, preferably between about 8 micrometres and about 50 micrometres, and more preferably between about 8 micrometres and about 39 micrometres.

The resistive heating mesh may cover an area of less than or equal to about 25 square millimetres. The resistive heating mesh may be rectangular. The resistive heating mesh may be square. The resistive heating mesh may have dimensions of about 5 millimetres by about 2 millimetres.

The electrically conductive filaments may comprise any suitable electrically conductive material. Suitable materials include but are not limited to: semiconductors such as doped ceramics, electrically "conductive" ceramics (such as, for example, molybdenum disilicide), carbon, graphite, metals, metal alloys and composite materials made of a ceramic material and a metallic material. Such composite materials may comprise doped or undoped ceramics. Examples of suitable doped ceramics include doped silicon carbides. Examples of suitable metals include titanium, zirconium, tantalum and metals from the platinum group. Examples of suitable metal alloys include stainless steel, constantan, nickel-, cobalt-, chromium-, aluminium-titanium- zirconium-, hafnium-, niobium-, molybdenum-, tantalum-, tungsten-, tin-, gallium-, manganese- and iron-containing alloys, and super-alloys based on nickel, iron, cobalt, stainless steel, Timetal®, iron-aluminium based alloys and iron-manganese-aluminium based alloys. Timetal® is a registered trade mark of Titanium Metals Corporation. The filaments may be coated with one or more insulators. Preferred materials for the electrically conductive filaments are 304, 316, 304L, and 316L stainless steel, and graphite.

The electrical resistance of the resistive heating mesh is preferably between about 0.3 and about 4 Ohms. More preferably, the electrical resistance of the mesh is between about 0.5 and about 3 Ohms, and more preferably about 1 Ohm.

In embodiments in which the first electric heater comprises a resistive heating coil, the pitch of the coil is preferably between about 0.5 millimetres and about 1.5 millimetres, and most preferably about 1.5 millimetres. The pitch of the coil means the spacing between adjacent turns of the coil. The coil may comprise fewer than six turns, and preferably has fewer than five turns. The coil may be formed from an electrically resistive wire having a diameter of between about 0.10 millimetres and about 0.15 millimetres, preferably about 0.125 millimetres. The electrically resistive wire is preferably formed of 904 or 301 stainless steel. Examples of other suitable metals include titanium, zirconium, tantalum and metals from the platinum group. Examples of other suitable metal alloys include, Constantan, nickel-, cobalt-, chromium-, aluminium- titanium-zirconium-, hafnium-, niobium-, molybdenum-, tantalum-, tungsten-, tin-, gallium-, manganese- and iron-containing alloys, and super-alloys based on nickel, iron, cobalt, stainless steel, Timetal®, iron-aluminium based alloys and iron-manganese-aluminium based alloys. The resistive heating coil may also comprise a metal foil, such as an aluminium foil, which is provided in the form of a ribbon.

In embodiments in which the aerosol-generating system comprises a second electric heater provided on an outer surface of the aerosol-generating device, the second electric heater may comprise any of the heaters described herein with respect to the first electric heater.

The second electric heater may comprise a heating film, such as a flexible heating film. Advantageously, a heating film, and in particular a flexible heating film, may facilitate conformation of the second electric heater to the shape of the outer surface of the aerosol-generating device.

The power supply may comprise a battery. For example, the power supply may be a nickel-metal hydride battery, a nickel cadmium battery, or a lithium based battery, for example a lithium-cobalt, a lithium-iron-phosphate or a lithium-polymer battery. The power supply may alternatively be another form of charge storage device such as a capacitor. The power supply may require recharging and may have a capacity that allows for the storage of enough energy for use of the aerosol-generating device with more than one cartridge assembly.

The invention is further described, by way of example only, with reference to the accompanying drawings in which:
Figure 1 is a perspective view of an aerosol-generating system according to a first embodiment of the present invention, with the cartridge separated from the aerosol-generating device;
Figure 2 is a perspective view of the aerosol-generating system of Figure 1, with the cartridge received on the aerosol-generating device;
Figure 3 is a cross-sectional view of the aerosol-generating device of Figure 1;
Figure 4 is a cross-sectional view of the aerosol-generating system of Figure 1, with the cartridge received on the aerosol-generating device;
Figure 5 is a perspective view of an aerosol-generating system according to a second embodiment of the present invention;
Figure 6 is a perspective view of an aerosol-generating system according to a third embodiment of the present invention;
Figure 7 is a perspective view of an aerosol-generating system according to a fourth embodiment of the present invention;
Figure 8 is a cross-sectional view of the aerosol-generating device of Figure 7;
Figure 9 is a cross-sectional view of the aerosol-generating system of Figure 7, with the cartridge received on the aerosol-generating device;
Figure 10 is a perspective view of a cartridge according to a fifth embodiment of the present invention; and
Figure 11 is a perspective view of a cartridge according to sixth embodiment of the present invention.

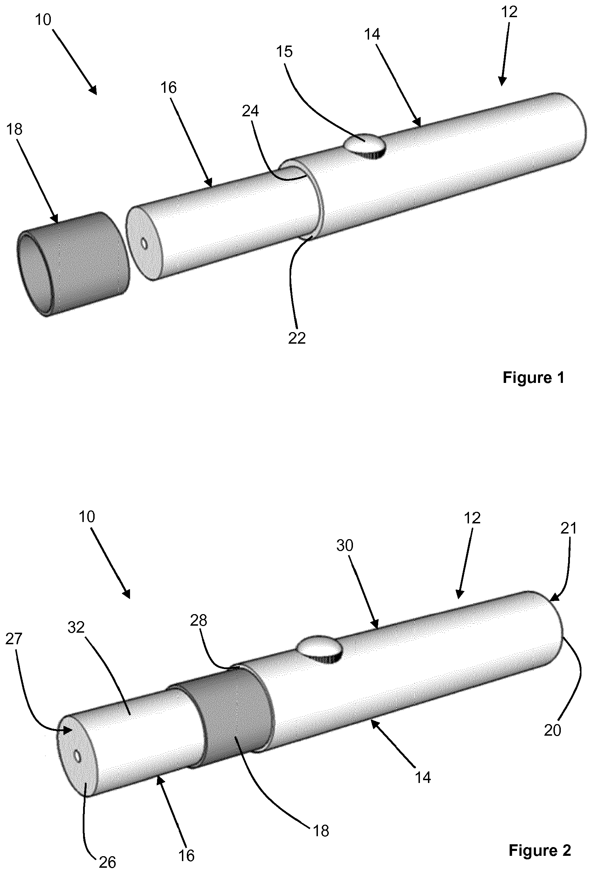

Figures 1 and 2 show perspective views of an aerosol-generating system 10 according to a first embodiment of the present invention. The aerosol-generating system 10 comprises an aerosol-generating device 12 comprising a power supply section 14 and a vaporiser section 16. The power supply section 14 comprises a button 15 for activating the aerosol-generating device 12.

The power supply section 14 comprises a first end 20 defining a first end 21 of the aerosol-generating system and a second end 22. The vaporiser section 16 comprises a first end 24 configured for removable attachment to the second end 22 of the power supply section 14, and a second end 26 defining a second end 27 of the aerosol-generating system 10. A diameter of the second end 22 of the power supply section 14 is larger than a diameter of the first end 24 of the vaporiser section 16 so that a portion of the second end 22 of the power supply section 14 forms a flange 28.

The aerosol-generating system 10 further comprises a cartridge 18 configured to be received on an outer surface 30 of the aerosol-generating device 12. In particular, the cartridge 18 has an annular shape and forms a sleeve configured to slide over an outer surface 32 of the vaporiser section 16 until an end of the cartridge 18 abuts the flange 28, as shown in Figure 2.

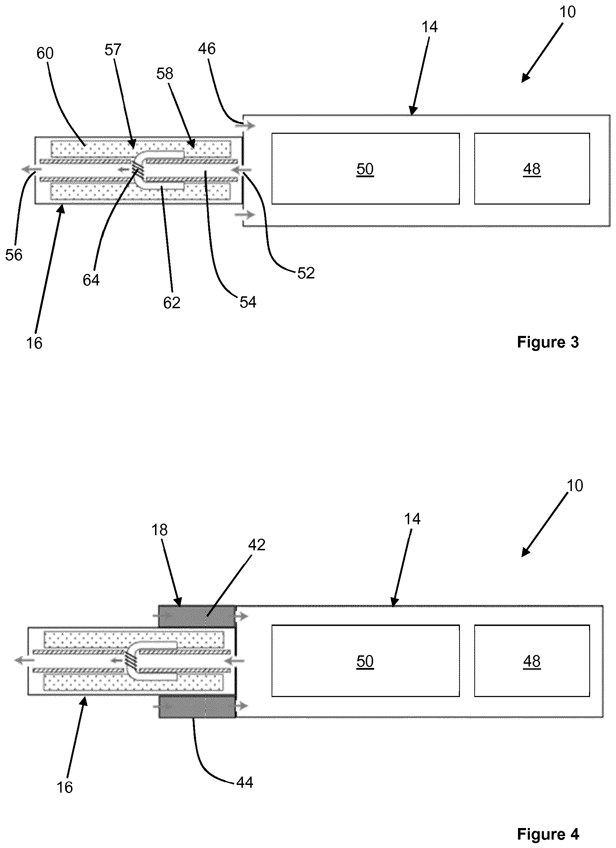

Figure 3 shows a cross-sectional view of the aerosol-generating device 12. The power supply section 14 defines a device air inlet 46 for admitting air into the power supply section 14, a controller 48 and a power supply 50. The device air inlet 46 is positioned on the flange 28.

The vaporiser section 16 comprises a vaporiser air inlet 52 for receiving air from the power supply section 14, an airflow passage 54 in fluid communication with the vaporiser air inlet 52 at its upstream end, and a device air outlet 56 in fluid communication with the downstream end of the airflow passage 54.

The vaporiser section 16 further comprises a liquid storage section 57 comprising a liquid aerosol-forming substrate 58 sorbed into an annular porous carrier material 60 positioned outside of the airflow passage 54. A liquid transfer element 62 comprising a capillary wick has first and second ends positioned in contact with the porous carrier material 60 and a central portion positioned within the airflow passage 54. Liquid aerosol-forming substrate 58 is wicked by capillary action along the capillary wick from the porous carrier material 60 to the central portion of the capillary wick.

The vaporiser section 16 also comprises a first electric heater 64 comprising a resistive heating coil wound around the central portion of the capillary wick. During operation of the aerosol-generating system 10, the controller 48 controls a supply of electrical energy from the power supply 50 to the first electric heater 64 to heat and vaporise liquid aerosol-forming substrate 58 from the central portion of the capillary wick.

When the cartridge 18 is received on the aerosol-generating device 12, as shown in Figure 4, the cartridge 18 abuts the flange 28 and overlies the device air inlet 46. The cartridge 18 comprises a cartridge aerosol-forming substrate 42 wrapped in a porous wrapper 44 so that, during use, air may flow through the cartridge 18 and into the device air inlet 46.

During operation of the aerosol-generating system 10, air is drawn into the aerosol-generating system 10 through the cartridge 18 where volatile compounds from the cartridge aerosol-forming substrate 42 are entrained in the airflow. The airflow then flows through the device air inlet 46, through the vaporiser air inlet 52 and into the airflow passage 54 where vaporised liquid aerosol-forming substrate 58 is entrained in the airflow. The airflow then flows out of the aerosol-generating system 10 through the device air outlet 56 to deliver to the user the vaporised liquid aerosol-forming substrate 58 and the volatile compounds from the cartridge aerosol-forming substrate 42.

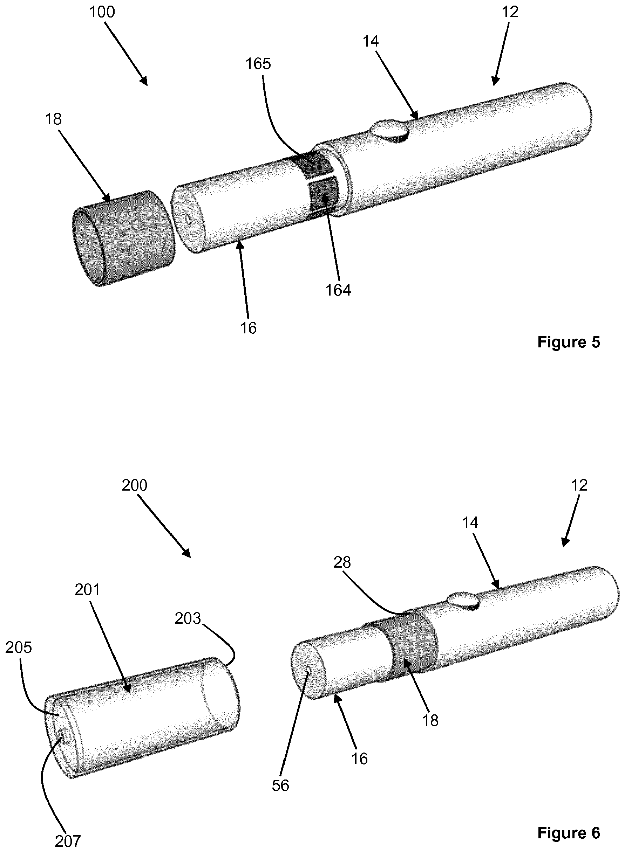

Figure 5 shows an aerosol-generating system 100 according to a second embodiment of the present invention. The aerosol-generating system 100 is substantially the same as the aerosol-generating system 10 shown in Figures 1 to 4, and like reference numerals are used to designate like parts.

The aerosol-generating system 100 differs only by the addition of a second electric heater 164 to the outer surface 32 of the vaporiser section 16 of the aerosol-generating device 12. The second electric heater 164 comprises a plurality of discrete flexible film heaters 165. During use, the cartridge 18 overlies the second electric heater 164 and the controller controls a supply of electrical energy from the power supply to the second electric heater 164 to heat the cartridge 18. Heating the cartridge 18 advantageously facilitates the release of volatile compounds from the cartridge aerosol-forming substrate.

Figure 6 shows an aerosol-generating system 200 according to a third embodiment of the present invention. The aerosol-generating system 200 is substantially the same as the aerosol-generating systems 10 and 100 shown in Figures 1 to 5, and like reference numerals are used to designate like parts.

The aerosol-generating system 200 differs only by the addition of a removable cover 201. The removable cover 201 is configured to slide over the vaporiser section 16 and the cartridge 18 to cover the cartridge 18 during use of the aerosol-generating system 200. An upstream end 203 of the removable cover 201 abuts the flange 28 when the removable cover 201 is received on the aerosol-generating device 12. A downstream end 205 of the removable cover 201 is closed and comprises a cover air outlet 207 configured to overlie the device air outlet 56. During use, the removable cover 201 is retained on the aerosol-generating device 12 by an interference fit.

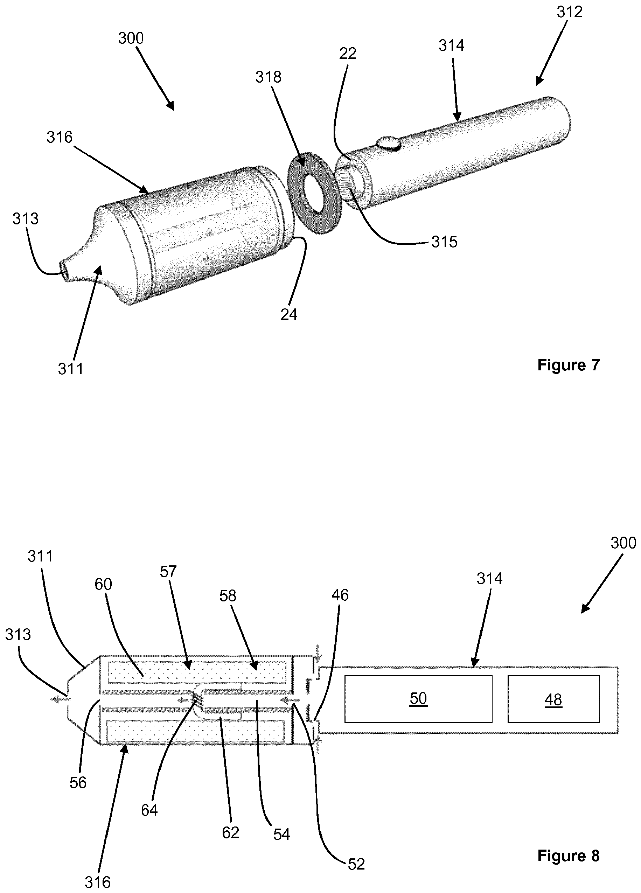

Figures 7 to 9 show an aerosol-generating system 300 according to a fourth embodiment of the present invention. The aerosol-generating system 300 is similar to the aerosol-generating system 10 shown in Figures 1 to 4, and like reference numerals are used to designate like parts.

The aerosol-generating system 300 comprises an aerosol-generating device 312 having a power supply section 314 and a vaporiser section 316 configured for removable attachment to each other by a screw connection. The power supply section 314 comprises a male screw connector 315 configured to attach to a female screw connector on the vaporiser section 316. The aerosol-generating system 300 is configured so that, when the male screw connector 315 is fully inserted into the female screw connector, the second end 22 of the power supply section 314 is spaced apart from the first end 24 of the vaporiser section 316 to define the device air inlet 46 therebetween.

The aerosol-generating system 300 comprises a cartridge 318 in the form of an annular ring. The cartridge 318 is configured to be received on the male screw connector 315 and sandwiched between the second end 22 of the power supply section 314 and the first end 24 of the vaporiser section 316 so that the cartridge 318 overlies the device air inlet 46.

The vaporiser section 316 further comprises a mouthpiece 311 at the second end of the vaporiser section, the mouthpiece 311 comprising a mouthpiece air outlet 313 in fluid communication with the device air outlet.

The operation of the aerosol-generating system 300 is substantially the same as the operation of the aerosol-generating system 10 shown in Figures 1 to 4. During use, air is drawn into the aerosol-generating system 300 through the cartridge 318 where volatile compounds from the cartridge aerosol-forming substrate 342 are entrained in the airflow. The airflow then flows through the device air inlet 46, through the vaporiser air inlet 52 and into the airflow passage 54 where vaporised liquid aerosol-forming substrate 58 is entrained in the airflow. The airflow then flows out of the aerosol-generating system 300 through the device air outlet 56 and the mouthpiece air outlet 313 to deliver to the user the vaporised liquid aerosol-forming substrate 58 and the volatile compounds from the cartridge aerosol-forming substrate 342.

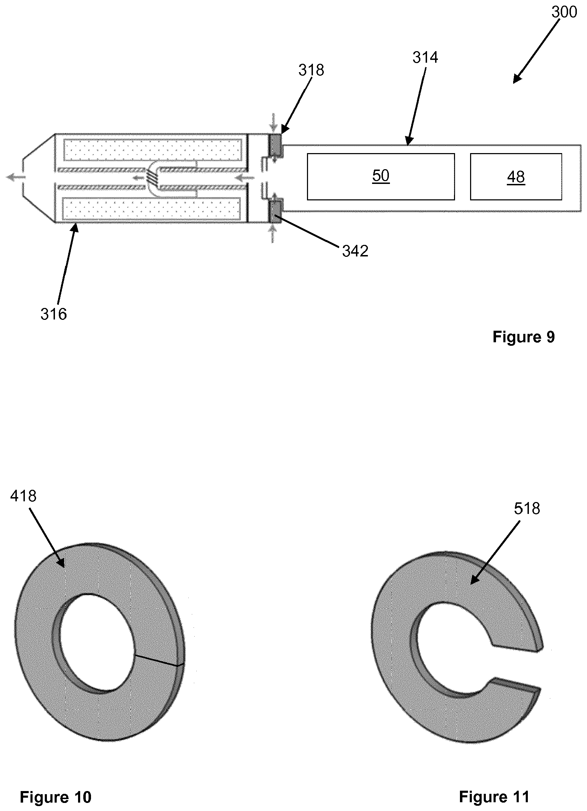

Figure 10 shows a cartridge 418 according to a fifth embodiment of the present invention. The cartridge 418 is substantially the same as the cartridge 318 shown in Figures 7 to 9, except the cartridge 418 has a split-ring shape, which may facilitate engagement and disengagement of the cartridge 418 with an aerosol-generating device. The cartridge 418 can be used with the aerosol-generating device 312 shown in Figures 7 to 9.

Figure 11 shows a cartridge 518 according to a sixth embodiment of the present invention. The cartridge 518 is substantially the same as the cartridge 318 shown in Figures 7 to 9, except the cartridge 518 has a C-shape, which may facilitate engagement and disengagement of the cartridge 518 with an aerosol-generating device. The cartridge 518 can be used with the aerosol-generating device 312 shown in Figures 7 to 9.

## Claims

1. An aerosol-generating system (10) comprising:
a cartridge (18) comprising a cartridge aerosol-forming substrate (42), the cartridge (18) having an annular shape; and
an aerosol-generating device (12) having a first end (21), a second end (27) and a length extending between the first end (21) and the second end (27), the aerosol-generating device (12) comprising:
a liquid storage section (57) comprising a liquid aerosol-forming substrate (58) positioned within the liquid storage section (57);
a first electric heater (64) configured to heat liquid aerosol-forming substrate (58) from the liquid storage section (57) during use of the aerosol-generating system (10);
a power supply section (14) comprising a power supply (50) and a controller (48) for controlling a supply of electrical power from the power supply (50) to the first electric heater (64);
a device air inlet (46) positioned between the first end (21) and the second end (27) of the aerosol-generating device (12); and
a device air outlet (56) positioned at the second end (27) of the aerosol-generating device (12);
wherein the power supply section (14) has a first end (20) forming the first end (21) of the aerosol-generating device (12) and a second end (22), wherein the liquid storage section (57) has a first end (24) configured for removable attachment to the second end (22) of the power supply section (14) and a second end (26) forming the second end (27) of the aerosol-generating device (12);
wherein an outer surface (32) of the aerosol-generating device (12) forms a flange (28), wherein one of the first end (24) of the liquid storage section (57) and the second end (22) of the power supply section (14) forms the flange (28) when the liquid storage section (57) is attached to the power supply section (14); and
wherein the aerosol-generating device (12) is configured to receive the cartridge (18) on the aerosol-generating device (12) so that the cartridge (18) is received against the flange (28) when the cartridge (18) is received on the aerosol-generating device (12), and wherein the device air inlet (46) is positioned proximate the flange (28) so that the cartridge (18) at least partially overlies the device air inlet (46) when the cartridge (18) is received against the flange (28).

2. An aerosol-generating system (10) according to claim 1, wherein the device air inlet (46) is positioned on the flange (28) so that an end of the cartridge (18) abuts the device air inlet (46) when the cartridge (18) is received against the flange (28).

3. An aerosol-generating system (10) according to claim 1, wherein the device air inlet (46) is positioned on the outer surface (32) of the aerosol-generating device (12) adjacent the flange (28) so that an inner surface of the cartridge (18) at least partially overlies the device air inlet (46) when the cartridge (18) is received against the flange (28).

4. An aerosol-generating system (100) according to any preceding claim, further comprising a second electric heater (164) provided on an outer surface (32) of the aerosol-generating device (12), wherein the second electric heater (164) is positioned so that the cartridge (18) at least partially overlies the second electric heater (164) when the cartridge (18) is received on the aerosol-generating device (12), and wherein the controller (48) is configured to control a supply of electrical power from the power supply (50) to the second electric heater (164) when the cartridge (18) is received on the aerosol-generating device (12).

5. An aerosol-generating system (200) according to any preceding claim, further comprising a removable cover (201) configured to receive at least a portion of the aerosol-generating device (12), wherein the removable cover (201) is configured to overlie the cartridge (18) when the cartridge (18) is received on the aerosol-generating device (12) and when the removable cover (201) is engaged with the aerosol-generating device (12).

6. An aerosol-generating system (200) according to claim 5, wherein the removable cover (201) comprises a cover air outlet (207) configured for fluid communication with the device air outlet (56) when the removable cover (201) is engaged with the aerosol-generating device (12).

7. An aerosol-generating system (200) according to claim 5 or 6, wherein the removable cover (201) further comprises a cover air inlet configured for fluid communication with the cartridge (18) when the cartridge (18) is received on the aerosol-generating device (12) and when the removable cover (201) is engaged with the aerosol-generating device (12).

8. An aerosol-generating system (200) according to claim 5, 6 or 7, wherein a first end (203) of the removable cover (201) is configured to abut the flange (28) when the removable cover (201) is engaged with the aerosol-generating device (12).

9. An aerosol-generating system (10) according to any preceding claim, wherein the cartridge (18) forms a sleeve configured to slide onto a portion of the aerosol-generating device (12).

10. An aerosol-generating system (300) according to any of claims 1 to 8, wherein the liquid storage section (57) is configured for removable attachment to the power supply section (314) and wherein the cartridge (318) is configured to be received between the liquid storage section (57) and the power supply section (314).

11. An aerosol-generating system (300) according to claim 10, wherein the cartridge (318) forms an annular disc.

12. An aerosol-generating system (300) according to claim 11, wherein the annular disc is one of a ring shape, a split-ring shape, and a C-shape.

13. An aerosol-generating system (10) according to any preceding claim wherein the cartridge (18) comprises a porous wrapper (44) overlying at least a portion of the cartridge aerosol-forming substrate (42).

## Patentansprüche

1. Aerosolerzeugungssystem (10), aufweisend:
eine Patrone (18), die ein patroniertes aerosolbildendes Substrat (42) aufweist, wobei die Patrone (18) eine ringförmige Form aufweist; und
eine Aerosolerzeugungsvorrichtung (12) mit einem ersten Ende (21), einem zweiten Ende (27) und einer Länge, die sich zwischen dem ersten Ende (21) und dem zweiten Ende (27) erstreckt, wobei die Aerosolerzeugungsvorrichtung (12) aufweist:
einen Flüssigspeicherabschnitt (57), der ein flüssiges aerosolbildendes Substrat (58) aufweist, das innerhalb des Flüssigspeicherabschnitts (57) positioniert ist;
eine erste elektrische Heizvorrichtung (64), die zum Erwärmen eines flüssigen aerosolbildenden Substrats (58) aus dem Flüssigspeicherabschnitt (57) während des Gebrauchs des Aerosolerzeugungssystems (10) ausgelegt ist;
einen Stromversorgungsabschnitt (14), der eine Stromversorgung (50) und eine Steuerung (48) zum Regeln einer Versorgung von elektrischem Strom von der Stromversorgung (50) zu der ersten elektrischen Heizvorrichtung (64) aufweist;
einen Gerätelufteinlass (46), der zwischen dem ersten Ende (21) und dem zweiten Ende (27) der Aerosolerzeugungsvorrichtung (12) positioniert ist und
einen Geräteluftauslass (56), der an dem zweiten Ende (27) der Aerosolerzeugungsvorrichtung (12) positioniert ist;
wobei der Stromversorgungsabschnitt (14) ein erstes Ende (20), das das erste Ende (21) der Aerosolerzeugungsvorrichtung (12) ausbildet, und ein zweites Ende (22) hat, wobei der Flüssigspeicherabschnitt (57) ein erstes Ende (24), das zur abnehmbaren Befestigung an dem zweiten Ende (22) des Stromversorgungsabschnitts (14) ausgelegt ist, und ein zweites Ende (26), das das zweite Ende (27) der Aerosolerzeugungsvorrichtung (12) ausbildet, hat;
wobei eine Außenfläche (32) der Aerosolerzeugungsvorrichtung (12) einen Flansch (28) ausbildet, wobei eines des ersten Endes (24) des Flüssigspeicherabschnitts (57) und des zweiten Endes (22) des Stromversorgungsabschnitts (14) den Flansch (28) ausbildet, wenn der Flüssigspeicherabschnitt (57) an dem Stromversorgungsabschnitt (14) befestigt ist und
wobei die Aerosolerzeugungsvorrichtung (12) so ausgelegt ist, dass sie die Patrone (18) an der Aerosolerzeugungsvorrichtung (12) aufnimmt, sodass die Patrone (18) durch den Flansch (28) aufgenommen wird, wenn die Patrone (18) an der Aerosolerzeugungsvorrichtung (12) aufgenommen wird, und wobei der Gerätelufteinlass (46) nahe des Flansches (28) positioniert ist, sodass die Patrone (18) zumindest teilweise über dem Gerätelufteinlass (46) liegt, wenn die Patrone (18) durch den Flansch (28) aufgenommen wird.

2. Aerosolerzeugungssystem (10) nach Anspruch 1, wobei der Gerätelufteinlass (46) auf dem Flansch (28) positioniert ist, sodass ein Ende der Patrone (18) an dem Gerätelufteinlass (46) anliegt, wenn die Patrone (18) durch den Flansch (28) aufgenommen wird.

3. Aerosolerzeugungssystem (10) nach Anspruch 1, wobei der Gerätelufteinlass (46) an der Außenfläche (32) der Aerosolerzeugungsvorrichtung (12) neben dem Flansch (28) positioniert ist, sodass eine Innenfläche der Patrone (18) zumindest teilweise über dem Gerätelufteinlass (46) liegt, wenn die Patrone (18) durch den Flansch (28) aufgenommen wird.

4. Aerosolerzeugungssystem (100) nach einem der vorhergehenden Ansprüche, das ferner eine zweite elektrische Heizvorrichtung (164) aufweist, die an einer Außenfläche (32) der Aerosolerzeugungsvorrichtung (12) vorgesehen ist, wobei die zweite elektrische Heizvorrichtung (164) so positioniert ist, dass die Patrone (18) zumindest teilweise über der zweiten elektrischen Heizvorrichtung (164) liegt, wenn die Patrone (18) auf der Aerosolerzeugungsvorrichtung (12) aufgenommen wird, und wobei die Steuerung (48) so ausgelegt ist, dass sie eine Versorgung von elektrischem Strom von der Stromversorgung (50) zu der zweiten elektrischen Heizvorrichtung (164) regelt, wenn die Patrone (18) auf der Aerosolerzeugungsvorrichtung (12) aufgenommen wird.

5. Aerosolerzeugungssystem (200) nach einem der vorhergehenden Ansprüche, das ferner eine abnehmbare Abdeckung (201) aufweist, die so ausgelegt ist, dass sie zumindest einen Teil der Aerosolerzeugungsvorrichtung (12) aufnimmt, wobei die abnehmbare Abdeckung (201) so ausgelegt ist, dass sie über der Patrone (18) liegt, wenn die Patrone (18) auf der Aerosolerzeugungsvorrichtung (12) aufgenommen wird und wenn die abnehmbare Abdeckung (201) mit der Aerosolerzeugungsvorrichtung (12) in Eingriff steht.

6. Aerosolerzeugungssystem (200) nach Anspruch 5, wobei die abnehmbare Abdeckung (201) einen Abdeckluftauslass (207) aufweist, der für eine Fluidverbindung mit dem Geräteluftauslass (56) ausgelegt ist, wenn die abnehmbare Abdeckung (201) mit der Aerosolerzeugungsvorrichtung (12) in Eingriff steht.

7. Aerosolerzeugungssystem (200) nach Anspruch 5 oder 6, wobei die abnehmbare Abdeckung (201) ferner einen Abdecklufteinlass aufweist, der für eine Fluidverbindung mit der Patrone (18) ausgelegt ist, wenn die Patrone (18) auf der Aerosolerzeugungsvorrichtung (12) aufgenommen wird und wenn die abnehmbare Abdeckung (201) mit der Aerosolerzeugungsvorrichtung (12) in Eingriff steht.

8. Aerosolerzeugungssystem (200) nach Anspruch 5, 6 oder 7, wobei ein erstes Ende (203) der abnehmbaren Abdeckung (201) so ausgelegt ist, dass es an dem Flansch (28) anliegt, wenn die abnehmbare Abdeckung (201) mit der Aerosolerzeugungsvorrichtung (12) in Eingriff steht.

9. Aerosolerzeugungssystem (10) nach einem der vorhergehenden Ansprüche, wobei die Patrone (18) eine Hülse ausbildet, die so ausgelegt ist, dass sie auf einen Teil der Aerosolerzeugungsvorrichtung (12) gleitet.

10. Aerosolerzeugungssystem (300) nach einem der Ansprüche 1 bis 8, wobei der Flüssigspeicherabschnitt (57) zur abnehmbaren Befestigung an dem Stromversorgungsabschnitt (314) ausgelegt ist und wobei die Patrone (318) so ausgelegt ist, dass sie zwischen dem Flüssigspeicherabschnitt (57) und dem Stromversorgungsabschnitt (314) aufgenommen wird.

11. Aerosolerzeugungssystem (300) nach Anspruch 10, wobei die Patrone (318) eine ringförmige Scheibe ausbildet.

12. Aerosolerzeugungssystem (300) nach Anspruch 11, wobei die ringförmige Scheibe eine aus einer Ringform, einer zweigeteilten Form oder einer C-Form ist.

13. Aerosolerzeugungssystem (10) nach einem der vorhergehenden Ansprüche, wobei die Patrone (18) eine poröse Umhüllung (44) aufweist, die zumindest über einem Teil des patronisierten aerosolbildenden Substrats (42) liegt.

## Revendications

1. Système de génération d'aérosol (10) comprenant :
une cartouche (18) comprenant un substrat formant aérosol de la cartouche (42), la cartouche (18) ayant une forme annulaire ; et
un dispositif générateur d'aérosol (12) ayant une première extrémité (21), une deuxième extrémité (27) et une longueur s'étendant entre la première extrémité (21) et la deuxième extrémité (27), le dispositif générateur d'aérosol (12) comprenant :
une section de stockage de liquide (57) comprenant un substrat formant aérosol liquide (58) positionné dans la section de stockage de liquide (57) ;
un premier dispositif de chauffage électrique (64) configuré pour chauffer le substrat formant aérosol liquide (58) de la section de stockage de liquide (57) pendant l'utilisation du système de génération d'aérosol (10) ;
une section d'alimentation électrique (14) comprenant une alimentation électrique (50) et un contrôleur (48) pour contrôler une alimentation en énergie électrique de l'alimentation électrique (50) au premier dispositif de chauffage électrique (64) ;
une entrée d'air du dispositif (46) positionnée entre la première extrémité (21) et la deuxième extrémité (27) du dispositif générateur d'aérosol (12) ; et
une sortie d'air du dispositif (56) positionnée dans la deuxième extrémité (27) du dispositif générateur d'aérosol (12) ;
dans lequel la section d'alimentation électrique (14) a une première extrémité (20) formant la première extrémité (21) du dispositif générateur d'aérosol (12) et une deuxième extrémité (22), dans lequel la section de stockage de liquide (57) a une première extrémité (24) configurée pour une fixation amovible à la deuxième extrémité (22) de la section d'alimentation électrique (14) et une deuxième extrémité (26) formant la deuxième extrémité (27) du dispositif générateur d'aérosol (12) ;
dans lequel une surface extérieure (32) du dispositif générateur d'aérosol (12) forme une bride (28), dans lequel l'une de la première extrémité (24) de la section de stockage de liquide (57) et la deuxième extrémité (22) de la section d'alimentation électrique (14) forme la bride (28) lorsque la section de stockage de liquide (57) est fixée à la section d'alimentation (14) ; et
dans lequel le dispositif générateur d'aérosol (12) est configuré pour recevoir la cartouche (18) sur le dispositif générateur d'aérosol (12) de telle sorte que la cartouche (18) soit reçue contre la bride (28) lorsque la cartouche (18) est reçue sur le dispositif générateur d'aérosol (12), et dans lequel l'entrée d'air du dispositif (46) est positionnée à proximité de la bride (28) de telle sorte que la cartouche (18) recouvre au moins partiellement l'entrée d'air du dispositif (46) lorsque la cartouche (18) est reçue contre la bride (28).

2. Système de génération d'aérosol (10) selon la revendication 1, dans lequel l'entrée d'air du dispositif (46) est positionnée sur la bride (28) de telle sorte qu'une extrémité de la cartouche (18) bute contre l'entrée d'air du dispositif (46) lorsque la cartouche (18) est reçue contre la bride (28).

3. Système de génération d'aérosol (10) selon la revendication 1, dans lequel l'entrée d'air du dispositif (46) est positionnée sur la surface extérieure (32) du dispositif générateur d'aérosol (12) adjacent à la bride (28) de telle sorte qu'une surface intérieure de la cartouche (18) recouvre au moins partiellement l'entrée d'air du dispositif (46) lorsque la cartouche (18) est reçue contre la bride (28).

4. Système de génération d'aérosol (100) selon l'une quelconque des revendications précédentes, comprenant en outre un deuxième dispositif de chauffage électrique (164) fourni sur une surface extérieure (32) du dispositif générateur d'aérosol (12), dans lequel le deuxième dispositif de chauffage électrique (164) est positionné de telle sorte que la cartouche (18) recouvre au moins partiellement le deuxième dispositif de chauffage électrique (164) lorsque la cartouche (18) est reçue sur le dispositif générateur d'aérosol (12), et dans lequel le contrôleur (48) est configuré pour commander une alimentation en énergie électrique de l'alimentation électrique (50) au deuxième dispositif de chauffage électrique (164) lorsque la cartouche (18) est reçue sur le dispositif générateur d'aérosol (12).

5. Système de génération d'aérosol (200) selon l'une quelconque revendication précédente, comprenant en outre un couvercle amovible (201) configuré pour recevoir au moins une partie du dispositif générateur d'aérosol (12), dans lequel le couvercle amovible (201) est configuré pour recouvrir la cartouche (18) lorsque la cartouche (18) est reçue sur le dispositif générateur d'aérosol (12) et lorsque le couvercle amovible (201) est engagé avec le dispositif générateur d'aérosol (12).

6. Système de génération d'aérosol (200) selon la revendication 5, dans lequel le couvercle amovible (201) comprend une sortie d'air du couvercle (207) configurée pour la communication fluidique avec la sortie d'air du dispositif (56) lorsque le couvercle amovible (201) est engagé avec le dispositif générateur d'aérosol (12) .

7. Système de génération d'aérosol (200) selon la revendication 5 ou 6, dans lequel le couvercle amovible (201) comprend en outre une entrée d'air du couvercle configurée pour la communication fluidique avec la cartouche (18) lorsque la cartouche (18) est reçue sur le dispositif générateur d'aérosol (12) et lorsque le couvercle amovible (201) est engagé avec le dispositif générateur d'aérosol (12).

8. Système de génération d'aérosol (200) selon la revendication 5, 6 ou 7, dans lequel une première extrémité (203) du couvercle amovible (201) est configurée pour venir en butée contre la bride (28) lorsque le couvercle amovible (201) est engagé avec le dispositif générateur d'aérosol (12).

9. Système de génération d'aérosol (10) selon l'une quelconque des revendications précédentes, dans lequel la cartouche (18) forme une gaine configurée pour glisser sur une partie du dispositif générateur d'aérosol (12).

10. Système de génération d'aérosol (300) selon l'une quelconque des revendications 1 à 8, dans lequel la section de stockage de liquide (57) est configurée pour une fixation amovible à la section d'alimentation électrique (314) et dans laquelle la cartouche (318) est configurée pour être reçue entre la section de stockage de liquide (57) et la section d'alimentation électrique (314).

11. Système de génération d'aérosol (300) selon la revendication 10, dans lequel la cartouche (318) forme un disque annulaire.

12. Système de génération d'aérosol (300) selon la revendication 11, dans lequel le disque annulaire a une forme d'anneau, une forme d'anneau fendu et une forme en C.

13. Système de génération d'aérosol (10) selon l'une quelconque des revendications précédentes, dans lequel la cartouche (18) comprend une enveloppe poreuse (44) recouvrant au moins une partie du substrat formant aérosol de la cartouche (42).
